# EUROPEAN PATENT SPECIFICATION

(11) **EP 1 554 279 B1**
(45) Date of publication and mention of the grant of the patent: **28.11.2007**
(21) Application number: 03755542.2
(22) Date of filing: 21.10.2003
(51) Int. Cl.: C07D 487/04

(54) **PROCESS FOR THE PREPARATION OF ZALEPLON**
VERFAHREN ZUR HERSTELLUNG VON ZALEPLON
PROCEDE DE PREPARATION DE ZALEPLON

(30) Priority: 25.10.2002 CZ 20023575
(43) Date of publication of application: 20.07.2005
(73) Proprietor: Zentiva, a.s., 102 37 Praha 10 (CZ)
(72) Inventor: RADL, Stanislav, 143 00 Praha 2 (CZ)
(74) Representative: Jirotkova, Ivana
(86) International application number: PCT/CZ2003/000057
(87) International publication number: WO 2004/037824

(56) References cited:
- WO-A-02/100828
- US-A1- 2002 072 605

## Description

### Technical Field

The invention concerns a new method of production of *N*-(3-(3-cyanopyrazolo[1,5-*a*]pyrimidin-7-yl)phenyl)-*N*-ethylacetamide, known under the INN (International Non-Proprietary Name) zaleplon, of formula I

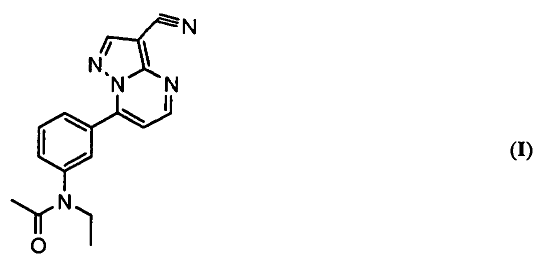

The aforesaid drug is an important representative of CNS chemotherapeutics, wherein it is used mainly as an anxiolytic, antiepileptic, or sedative/hypnotic drug.

### Background Art

Zaleplon is produced according to published US patents Nos. 4,626,538 and 5,714,607 via reaction of *N*-[3-(3-dimethylamino-acryloyl)-phenyl]-*N*-ethyl-acetamide of formula II 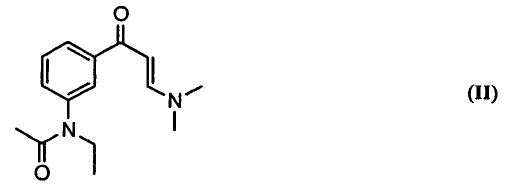 with 5-amino-1*H*-pyrazol-4-carbonitrile of formula III

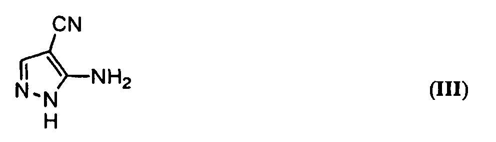

The reaction was performed in the original US patent No. 4,626,538 in anhydrous acetic acid. Later, it was discovered that the reaction is faster and the resulting product is purer if the reaction is carried out in aqueous acetic acid. This procedure is described in US patent No. 5,714,607.

Other process of (I) preparation is disclosed in US 2002/072605, in which compound (II) is formed from the corresponding acetanilide using sodium hydride and ethyliodide in DMF and the reaction mixture is worked-up by dilution with water. The formed mixture is without isolation of (II) treated with a solution of (III) in aqueous DMF in the presence of hydrochloric acid to form (I).

According to the patent application WO 02/100828 the reaction of the compound (II) with (I) is carried out in water and a water miscible solvent free of carboxylic groups under acidic conditions.

This invention describes a new, improved method of production of *N*-(3-(3-cyanopyrazolo[1,5-*a*]pyrimidin-7-yl)phenyl)-*N*-ethylacetamide (zaleplon).

### Disclosure of Invention

The subject matter of the invention is a new, improved method of production of *N*-(3-(3-cyanopyrazolo[1,5-*a*]pyrimidin-7-yl)phenyl)-*N*-ethylacetamide (zaleplon). The whole procedure is based on the surprising finding that it is advantageous to perform the reaction of *N*-[3-(3-dimethylamino-acryloyl)-phenyl]-*N*-ethyl-acetamide (II) with 5-amino-1*H*-pyrazol-4-carbonitrile (III) in numerous organic solvents or alternatively in their mixtures with water with a number of organic and inorganic acids being used as catalysts.

The essence of the invention consists in reacting *N*-[3-(3-dimethylamino-acryloyl)-phenyl]-*N*-ethyl-acetamide of formula II 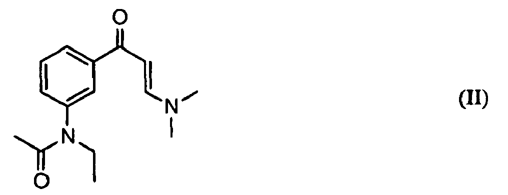 with 5-amino-1*H*-pyrazol-4-carbonitrile of formula III 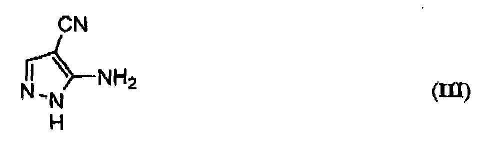 in a medium comprising a solution of hydrochloric or hydrobromic acid in a C1-C5 alcohol, or in a C4-C6 aliphatic or cyclic ether, or in a C2-C5 alkoxyalcohol, or in a 5- to 6-membered aromatic heterocyclic solvent, containing 1-2 oxygen atoms, or in a mixture thereof with water, with the proviso that no water is added to the mixture of the substances of formulae II and III before the reaction is started by the acid.
It has been shown that likewise as when aqueous acetic acid or aqueous formic acid are used, the reaction time gets significantly shorter when the above solvents and the above acidic catalysts are used. Moreover, in some cases, better yields and/or higher purity of the crude product were achieved.

In a usual embodiment, the two starting substances were mixed with a suitable solvent at laboratory temperature. The quantity of the used solvent can be selected from a wide range so that after the reaction is complete and cooled down, crystals of the product fall out in high yields. The reaction mixture was either a homogenous solution or a suspension. A suitable acidic catalyst was added to the mixture created in this way and the mixture was stirred at a temperature from 25 °C to the boiling point of the used solvent. After the reaction was completed, the mixture was cooled down; after crystals fell out, the mixture was left to sit for several hours at 5 up to 10°C and, subsequently, the crude product was sucked off. In some cases, the reaction mixture was mixed with a suitable co-solvent after it cooled down and the product was filtered after cooling down.

A number of solvents turned out to be suitable. The use of methanol, ethanol, propanol or 2-propanol turned out to be especially advantageous as high yields of highly pure crude product were obtained. The crude product provided a product with high HPLC purity, usually higher than 99.5% (read from areas of individual peaks) after a single crystallization from a suitable solvent. However, the reaction is not limited only to the environment of hydroxylated solvents; it can be performed in a number of other solvents, for example tetrahydrofuran, dioxane, dimethoxyethane, etc.; these can be used either alone or in admixtures with the above-mentioned hydroxylated solvents, or in their mixtures with water. Presence of 20 to 80 % of water is preferable.

In order for the reaction to proceed successfully, presence of acidic catalysts is necessary. Instead of aqueous acids, solutions of acids in suitable solvents can be used, for example, solutions of hydrogen chloride or hydrogen bromide in various solvents, C1-C5 alcohols being preferable.

The invention is explained in more detail in the following examples. The examples, which illustrate preferred alternatives of production of zaleplon according to the invention, have a purely illustrative character and do not limit the extent of the invention in any respect.

### Examples

### Example 1

A mixture of compound II (2.6 g, 10 mmol) and nitrile III (1.1 g, 10.2 mmol) in ethanol (30 ml) was dissolved at lab temperature and then, a saturated solution of hydrogen chloride in ethanol (1 ml) was added. The resulting mixture was boiled for 2 hours using a reflux condenser and, then, cooled down to 5 to 10 °C and the precipitated crystals were sucked off, washed with water and air dried. 2.6 g of crude product (85%), m.p. 184-188 °C were obtained. After crystallization, 2.4 g (79%) of crystals were obtained with m.p. 187-188 °C.

### Example 2

Following the procedure described in Example 1 wherein the reaction mixture was stirred for 2 days at lab temperature, a similar amount of a product was obtained with m.p. 182-186 °C. After crystallization from methanol, 72% of crystals were obtained with m.p. 185-187 °C.

### Example 3

A mixture of compound II (2.6 g, 10 mmol) and nitrile III (1.1 g, 10.2 mmol) in methanol (30 ml) is dissolved at lab temperature and, subsequently, concentrated hydrochloric acid was added (1 ml). The resulting mixture was boiled for 5 hours using a reflux condenser and, then, water was added (50 ml) and the mixture was cooled down to 5 to 10 °C. Crystals that fell out were sucked off, washed with water and air dried. 2.8 g of crude product were obtained (92%), m.p. 185-188 °C.

### Example 4

Following the procedure described in Example 1 wherein propanol was used as the solvent instead of ethanol, 86% of the product was obtained with m.p. 185-188 °C.

### Example 5

Following the procedure described in Example 1 wherein 2-propanol was used as the solvent instead of ethanol, 91% of the product was obtained with m.p. 184-187 °C.

### Example 6

Following the procedure described in Example 1 wherein 2-methoxyethanol was used as the solvent instead of ethanol, 76% of the product was obtained with m.p. 184-187 °C.

### Example 7

Following the procedure described in Example 1 wherein 2-ethoxyethanol was used as the solvent instead of ethanol, 79% of the product was obtained with m.p. 184-187 °C.

### Example 8

Following the procedure described in Example 1 wherein 70% of aqueous ethanol was used as the solvent instead of ethanol, 87% of the product was obtained with m.p. 185-188 °C.

### Example 9

Following the procedure described in Example 1 wherein 50% of aqueous ethanol was used as the solvent instead of ethanol, 69% of the product was obtained with m.p. 184-187°C.

### Example 10

Following the procedure described in Example 1 wherein 30% of aqueous ethanol was used as the solvent instead of ethanol, 72% of the product was obtained with m.p. 184-188 °C.

### Example 11

A mixture of compound II (0.26 g, 1 mmol) and nitrile III (0.11 g, 1.0 mmol) in tetrahydrofuran (3 ml) was dissolved at lab temperature and, subsequently, concentrated hydrochloric acid was added (1 ml). The resulting mixture was boiled for 2 hours using a reflux condenser and, then, water was added (5 ml) and the mixture was cooled down to 5 to 10 °C. Crystals that fell out were sucked off, washed with water and air dried. 0.25 g of crude product were obtained (82%), m.p. 184-187 °C.

### Example 12

Following the procedure described in Example 11 wherein dioxane was used as the solvent instead of tetrahydrofuran and hexane was added instead of water after the reaction was completed, 86% of the product was obtained with m.p. 183-187 °C

### Example 13

Following the procedure described in Example 1 wherein hydrobromic acid was used as the catalyst instead of hydrochloric acid, 81 % of the product was obtained with m.p. 184-188 °C.

### Example 14

Following the procedure described in Example 1 wherein 10% of sulfuric acid was used as the catalyst instead of hydrochloric acid, 82% of the product was obtained after thorough washing with water and drying with m.p. 184-187 °C.

### Example 15

Following the procedure described in Example 1 wherein 10% of orthophosphoric acid was used as the catalyst instead of hydrochloric acid and reaction time was increased to 8 hours, 65% of the product was obtained after thorough washing with water and drying with m.p. 182-186 °C.

### Example 16

Following the procedure described in Example 1 wherein perchloric acid was used as the catalyst instead of hydrochloric acid, 69% of the product was obtained after thorough washing with water and drying with m.p. 184-187 °C.

### Example 17

A mixture of compound II (0.26 g, 1 mmol) and nitrile III (0.11 g, 1.0 mmol) in ethanol (3 ml) was dissolved at lab temperature and, subsequently, acetic acid was added (1 ml). The resulting mixture was boiled for 3 hours using a reflux condenser and, then, it was cooled down to 5 to 10 °C. Crystals that fell out were sucked off, washed with water and air dried. 0.26 g of crude product were obtained (85%), m.p. 184-187 °C.

### Example 18

A mixture of compound II (0.26 g, 1 mmol) and nitrile III (0.11 g, 1.0 mmol) in methanol (3 ml) was dissolved at lab temperature and, subsequently, trifluoroacetic acid was added (0.1 ml). The resulting mixture was boiled for 1 hours using a reflux condenser and, then, it was cooled down to 5 to 10 °C. Crystals that fell out were sucked off, washed with water and air dried. 0.24 g of crude product were obtained (79%), m.p. 184-187 °C.

### Example 19

Following the procedure described in Example 1 wherein methanesulfonic acid was used as the catalyst instead of hydrochloric acid, 83% of the product was obtained after thorough washing with water and drying with m.p. 183-186 °C.

### Example 20

Following the procedure described in Example 1 wherein benzenesulfonic acid was used as the catalyst instead of hydrochloric acid, 66% of the product was obtained after thorough washing with water and drying with m.p. 183-186 °C.

### Example 21

Following the procedure described in Example 1 wherein 4-toluenesulfonic acid was used as the catalyst instead of hydrochloric acid, 59% of the product was obtained after thorough washing with water and drying with m.p. 184-187 °C.

## Claims

1. A method of production of *N*-(3-(3-cyanopyrazolo[1,5-*a*]pyrimidin-7-yl)phenyl)-*N-*ethylacetamide, known under the INN name zaleplon, of formula I 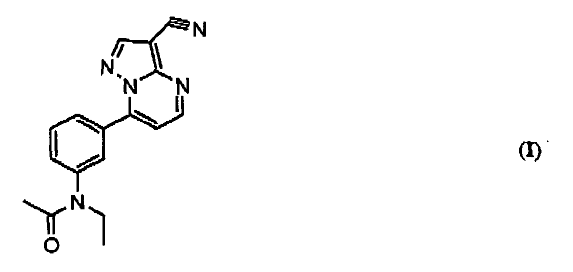 **characterized in that** *N*-[3-(3-dimethylamino-acryloyl)-phenyl]-*N*-ethyl-acetamide of formula II 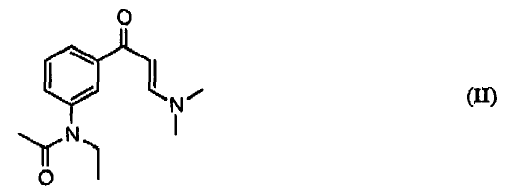 is reacted with 5-amino-1*H*-pyrazol-4-carbonitrile of formula III 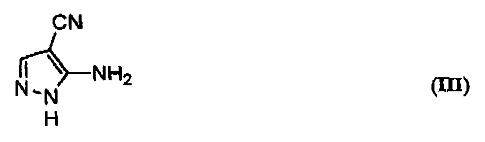 in a medium comprising a solution of hydrochloric or hydrobromic acid in a C1-C5 alcohol, or in a C4-C6 aliphatic or cyclic ether, or in a C2-C5 alkoxyalcohol, or in a 5- to 6-membered aromatic heterocyclic solvent, containing 1-2 oxygen atoms, or in a mixture thereof with water, with the proviso that no water is added to the mixture of the substances of formulae II and III before the reaction is started by the acid.

2. The method according to claim 1 **characterized in that** ethanol is used as the solvent.

3. The method according to claim 1 **characterized in that** methanol is used as the solvent.

4. The method according to claim 1 **characterized in that** propanol is used as the solvent.

5. The method according to claim 1 **characterized in that** 2-propanol is used as the solvent.

6. The method according to claim 1 **characterized in that** 2-methoxyethanol is used as the solvent.

7. The method according to claim 1 **characterized in that** 2-ethoxyethanol is used as the solvent.

8. The method according to claim 1 **characterized in that** tetrahydrofuran is used as the solvent.

9. The method according to claim 1 **characterized in that** dioxane is used as the solvent.

10. The method according to any of the preceding claims **characterized in that** water is used as the co-solvent, preferably final mixtures containing 20 to 80% of water being used.

## Patentansprüche

1. Verfahren zur Herstellung von *N*-[3-(3-Zyano-pyrazol[1,5-*a*]pyrimidin-7-yl)-phenyl]-*N-*ethyl-azetamid, bekannt unter der INN-Bezeichnung Zaleplon, der Formel I 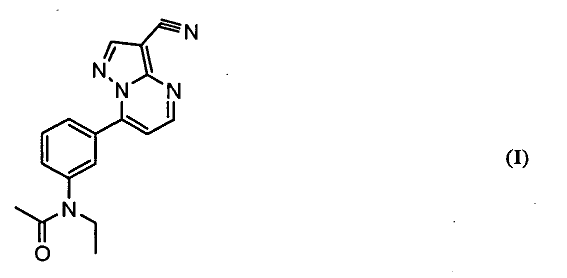 **dadurch gekennzeichnet, dass** man *N*-[3-(3-Dimethylamino-akryloyl)-phenyl]-*N*-ethyl-azetamid der Formel II 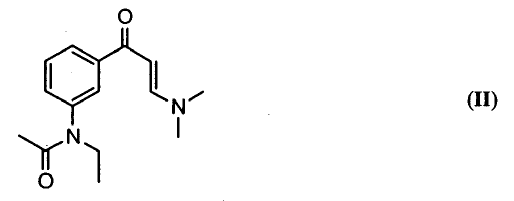 mit 5-Amino-1*H*-pyrazol-4-karbonitril der Formel III umsetzt 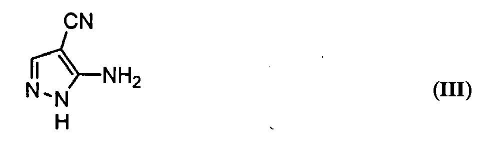 in einem Medium, enthaltend eine Lösung der Salzsäure oder Bromwasserstoffsäure im C1-C5-Alkohol, oder im aliphatischen, bzw. zyklischen C4-C6-Äther oder im C2-C5-Alkoxyalkohol, bzw. in einem 5- bis 6-gliedrigen aromatischen heterozyklischen Lösungsmittel, die 1-2 Sauerstoffatome enthält, bzw. deren Gemisch mit Wasser, unter der Bedingung, dass zu dem Gemisch der Stoffe den Formeln II und IIII kein Wasser vor Einleitung der Reaktion mit Säure zugegeben wird.

2. Verfahren nach Anspruch 1, **dadurch gekennzeichnet, dass** man als Lösungsmittel Äthanol verwendet.

3. Verfahren nach Anspruch 1, **dadurch gekennzeichnet, dass** man als Lösungsmittel Methanol verwendet.

4. Verfahren nach Anspruch 1, **dadurch gekennzeichnet, dass** man als Lösungsmittel Propanol verwendet.

5. Verfahren nach Anspruch 1, **dadurch gekennzeichnet, dass** man als Lösungsmittel 2-Propanol verwendet.

6. Verfahren nach Anspruch 1, **dadurch gekennzeichnet, dass** man als Lösungsmittel 2-Methoxyäthanol verwendet.

7. Verfahren nach Anspruch 1, **dadurch gekennzeichnet, dass** man als Lösungsmittel 2-Äthoxyäthanol verwendet.

8. Verfahren nach Anspruch 1, **dadurch gekennzeichnet, dass** man als Lösungsmittel Tetrahydrofuran verwendet.

9. Verfahren nach Anspruch 1, **dadurch gekennzeichnet, dass** man als Lösungsmittel Dioxan verwendet.

10. Verfahren nach einem der vorhergehenden Ansprüche, **dadurch gekennzeichnet, dass** man als Co-solvent Wasser verwendet, wobei vorteilhaft finale Mischungen, enthaltend 20 - 80% Wasser, verwendet werden.

## Revendications

1. Procédé de fabrication du *N*-(3-(3-cyano-pyrazolo[1,5-*a*]pyrimidin-7-yl)-phényl)-*N*-éthyl-acétamide connu par la désignation INN zaléplon, de formule I, 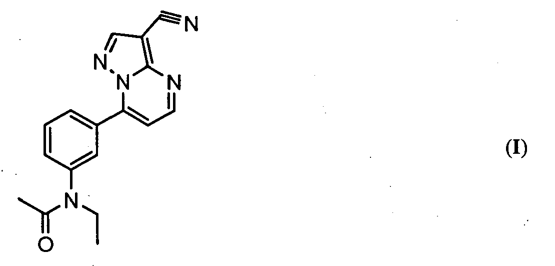 **caractérisé en ce que** l'on fait réagir le *N*-[3-(3-diméthylamino-acryloyl)-phényl]-*N*-éthyl-acétamide de formule II 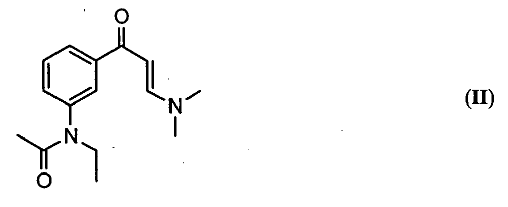 avec le 5-amino-1*H*-pyrazol-4-carbonitrile de formule III 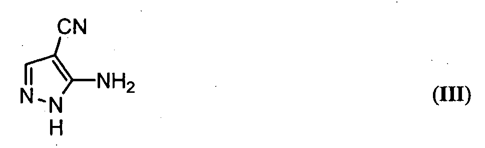 dans le milieu contenant la solution de l'acide chlorhydrique ou bromhydrique dans l'alcool C1-C5 ou bien dans l'éther aliphatique ou cyclique C4-C6 ou dans l'acoxyalcool C2-C5 ou bien dans un solvant aromatique hétérocyclique à 5-6 chaînons qui comprend 1-2 atomes d'oxygène, ou dans leur mélange avec l'eau, à la condition que l'eau ne soit pas ajoutée au mélange des substances de formules II et III avant l'ouverture de la réaction par l'acide.

2. Procédé selon la revendication 1, **caractérisé en ce que** l'on utilise l'éthanol comme le solvant.

3. Procédé selon la revendication 1, **caractérisé en ce que** l'on utilise le méthanol comme le solvant.

4. Procédé selon la revendication 1, **caractérisé en ce que** l'on utilise le propanol comme le solvant.

5. Procédé selon la revendication 1, **caractérisé en ce que** l'on utilise le 2-propanol comme le solvant.

6. Procédé selon la revendication 1, **caractérisé en ce que** l'on utilise le 2- méthoxyéthanol comme le solvant.

7. Procédé selon la revendication 1, **caractérisé en ce que** l'on utilise le 2-éthoxyéthanol comme le solvant.

8. Procédé selon la revendication 1, **caractérisé en ce que** l'on utilise le tétrahydrofurane comme le solvant.

9. Procédé selon la revendication 1, **caractérisé en ce que** l'on utilise le dioxane comme le solvant.

10. Procédé selon une quelconque des revendications précédentes, **caractérisé en ce que** l'on utilise l'eau comme le co-solvant, les mélanges finaux contenant entre 20 et 80 % de l'eau étant utilisés d'avantage.
